(19) Europäisches Patentamt / European Patent Office / Office européen des brevets

(11) **EP 2 046 411 B1**

(12) **EUROPEAN PATENT SPECIFICATION**

(45) Date of publication and mention of the grant of the patent:
**30.12.2015 Bulletin 2015/53**

(51) Int Cl.:
**A61L 31/14** *(2006.01)*    **B29C 47/24** *(2006.01)*

(21) Application number: **07809689.8**

(22) Date of filing: **19.06.2007**

(86) International application number:
**PCT/US2007/014316**

(87) International publication number:
**WO 2007/149457 (27.12.2007 Gazette 2007/52)**

(54) **FABRICATING A STENT WITH SELECTED PROPERTIES IN THE RADIAL AND AXIAL DIRECTIONS**

HERSTELLUNG EINES STENTS MIT AUSGEWÄHLTEN EIGENSCHAFTEN DER RADIALEN UND AXIALEN AUSRICHTUNG

FABRICATION D'UN STENT AVEC DES PROPRIÉTÉS CHOISIES DANS LES DIRECTIONS RADIALE ET AXIALE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **19.06.2006   US 471376**

(43) Date of publication of application:
**15.04.2009   Bulletin 2009/16**

(73) Proprietor: **Abbott Cardiovascular Systems Inc.**
**Santa Clara, CA 95054-2807 (US)**

(72) Inventors:
• **CAPEK, John**
  **Saratoga, California 95070 (US)**
• **GALE, David C.**
  **Kennesaw, G 30152 (US)**
• **HUANG, Bin**
  **Pleasanton, California 94566 (US)**
• **HOSSAINY, Syed Faiyaz Ahmed**
  **Fremont, California 94555 (US)**

(74) Representative: **ZBM Patents - Zea, Barlocci & Markvardsen**
**Plaza Catalunya, 1**
**08002 Barcelona (ES)**

(56) References cited:
**US-A1- 2003 187 158    US-A1- 2005 137 678**
**US-A1- 2006 020 330    US-A1- 2006 076 708**

**Description**

BACKGROUND OF THE INVENTION

Field of the Invention

This invention relates to methods of fabricating stents having selected mechanical properties.

Description of the State of the Art

This invention relates to radially expandable endoprostheses, which are adapted to be implanted in a bodily lumen. An "endoprosthesis" corresponds to an artificial device that is placed inside the body. A "lumen" refers to a cavity of a tubular organ such as a blood vessel.

A stent is an example of such an endoprosthesis. Stents are generally cylindrically shaped devices, which function to hold open and sometimes expand a segment of a blood vessel or other anatomical lumen such as urinary tracts and bile ducts. Stents are often used in the treatment of atherosclerotic stenosis in blood vessels. "Stenosis" refers to a narrowing or constriction of the diameter of a bodily passage or orifice. In such treatments, stents reinforce body vessels and prevent restenosis following angioplasty in the vascular system. "Restenosis" refers to the reoccurrence of stenosis in a blood vessel or heart valve after it has been treated (as by balloon angioplasty, stenting, or valvuloplasty) with apparent success.

The treatment of a diseased site or lesion with a stent involves both delivery and deployment of the stent. "Delivery" refers to introducing and transporting the stent through a bodily lumen to a region, such as a lesion, in a vessel that requires treatment. "Deployment" corresponds to the expanding of the stent within the lumen at the treatment region. Delivery and deployment of a stent are accomplished by positioning the stent about one end of a catheter, inserting the end of the catheter through the skin into a bodily lumen, advancing the catheter in the bodily lumen to a desired treatment location, expanding the stent at the treatment location, and removing the catheter from the lumen.

In the case of a balloon expandable stent, the stent is mounted about a balloon disposed on the catheter. Mounting the stent typically involves compressing or crimping the stent onto the balloon. The stent is then expanded by inflating the balloon. The balloon may then be deflated and the catheter withdrawn. In the case of a self-expanding stent, the stent may be secured to the catheter via a retractable sheath or a sock. When the stent is in a desired bodily location, the sheath may be withdrawn which allows the stent to self-expand.

The stent must be able to satisfy a number of mechanical requirements. First, the stent must be capable of withstanding the structural loads, namely radial compressive forces, imposed on the stent as it supports the walls of a vessel. Therefore, a stent must possess adequate radial strength. Radial strength, which is the ability of a stent to resist radial compressive forces, is due to strength and rigidity around a circumferential direction of the stent. Radial strength and rigidity, therefore, may also be described as, hoop or circumferential strength and rigidity.

Once expanded, the stent must adequately maintain its size and shape throughout its service life despite the various forces that may come to bear on it, including the cyclic loading induced by the beating heart. For example, a radially directed force may tend to cause a stent to recoil inward. Generally, it is desirable to minimize recoil.

In addition, the stent must possess sufficient flexibility to allow for crimping, expansion, and cyclic loading. Longitudinal flexibility is important to allow the stent to be maneuvered through a tortuous vascular path and to enable it to conform to a deployment site that may not be linear or may be subject to flexure. Finally, the stent must be biocompatible so as not to trigger any adverse vascular responses.

The structure of a stent is typically composed of scaffolding that includes a pattern or network of interconnecting structural elements often referred to in the art as struts or bar arms. The scaffolding can be formed from wires, tubes, or sheets of material rolled into a cylindrical shape. The scaffolding is designed so that the stent can be radially compressed (to allow crimping) and radially expanded (to allow deployment). A conventional stent is allowed to expand and contract through movement of individual structural elements of a pattern with respect to each other.

Additionally, a medicated stent may be fabricated by coating the surface of either a metallic or polymeric scaffolding with a polymeric carrier that includes an active or bioactive agent or drug. Polymeric scaffolding may also serve as a carrier of an active agent or drug.

Furthermore, it may be desirable for a stent to be biodegradable. In many treatment applications, the presence of a stent in a body may be necessary for a limited period of time until its intended function of, for example, maintaining vascular patency and/or drug delivery is accomplished. Therefore, stents fabricated from biodegradable, bioabsorbable, and/or bioerodable materials such as bioabsorbable polymers should be configured to completely erode only after the clinical need for them has ended.

In general, there are several important aspects in the mechanical behavior of polymers that affect stent design. Polymers tend to have lower strength than metals on a per unit mass basis. Therefore, polymeric stents typically have less circumferential strength and radial rigidity than metallic stents of the same or similar dimensions. Inadequate radial strength potentially contributes to a relatively high incidence of recoil of polymeric stents after implantation into vessels.

Another potential problem with polymeric stents is that their struts or bar arms can crack during crimping and expansion, especially for brittle polymers. The localized portions of the stent pattern subjected to substantial deformation tend to be the most vulnerable to failure. Furthermore, in order to have adequate mechanical strength, polymeric stents may require significantly thicker struts than a metallic stent, which results in an undesirably larger profile.

Conventional methods of constructing a stent from a polymer material involve extrusion of a polymer tube based on a single polymer or polymer blend and then laser cutting a pattern into the tube. US 2006/0076708 discloses methods of manufacturing a radially expandable stent, including axial elongation and radial expansion of a polymer tube.

Therefore, it would be desirable to have methods of making biodegradable polymeric stents that are both strong and flexible.

## SUMMARY OF THE INVENTION

The present invention provides methods for fabricating stents according to claim 1. Several embodiments are further described in the dependent claims.

## BRIEF DESCRIPTION OF THE DRAWINGS

FIG. 1 depicts a stents.
FIG. 2 depicts a tube.
FIGs. 3-4 depict blow-molding of a polymeric tube.
FIG. 5 depicts a schematic plot of the crystal nucleation rate and the crystal growth rate, and the overall rate of crystallization.
FIG. 6 depicts a tube showing an examplary axial dogbone sample.
FIG. 7 depicts a tube showing an exemplary radial dogbone sample.

## DETAILED DESCRIPTION OF THE INVENTION

The various embodiments of the present invention relate to methods of fabricating a polymeric stent that have selected mechanical properties along the axial direction or circumferential direction of the stent, or both. The present invention can be applied to devices including, but is not limited to, self-expandable stents, balloon-expandable stents, stent-grafts, and grafts (e.g., aortic grafts).

For the purposes of the present invention, the following terms and definitions apply:

The "glass transition temperature," $T_g$, is the temperature at which the amorphous domains of a polymer change from a brittle vitreous state to a solid deformable or ductile state at atmospheric pressure. In other words, the $T_g$ corresponds to the temperature where the onset of segmental motion in the chains of the polymer occurs. When an amorphous or semicrystalline polymer is exposed to an increasing temperature, the coefficient of expansion and the heat capacity of the polymer both increase as the temperature is raised, indicating increased molecular motion. As the temperature is raised the actual molecular volume in the sample remains constant, and so a higher coefficient of expansion points to an increase in free volume associated with the system and therefore increased freedom for the molecules to move. The increasing heat capacity corresponds to an increase in heat dissipation through movement. $T_g$ of a given polymer can be dependent on the heating rate and can be influenced by the thermal history of the polymer. Furthermore, the chemical structure of the polymer heavily influences the glass transition by affecting mobility.

"Stress" refers to force per unit area, as in the force acting through a small area within a plane. Stress can be divided into components, normal and parallel to the plane, called normal stress and shear stress, respectively. Tensile stress, for example, is a normal component of stress applied that leads to expansion (increase in length). In addition, compressive stress is a normal component of stress applied to materials resulting in their compaction (decrease in length). Stress may result in deformation of a material, which refers to a change in length. "Expansion" or "compression" may be defined as the increase or decrease in length of a sample of material when the sample is subjected to stress.

"Strain" refers to the amount of expansion or compression that occurs in a material at a given stress or load. Strain may be expressed as a fraction or percentage of the original length, i.e., the change in length divided by the original length. Strain, therefore, is positive for expansion and negative for compression.

"Modulus" may be defined as the ratio of a component of stress or force per unit area applied to a material divided by the strain along an axis of applied force that results from the applied force. For example, a material has both a tensile and a compressive modulus.

"Stress at peak" is the maximum tensile stress which a material will withstand prior to fracture. Stress at break can also be referred to as the tensile strength. The stress at break is calculated from the maximum load applied during a test divided by the original cross-sectional area.

"Stress at break" is the tensile stress of a material at fracture.

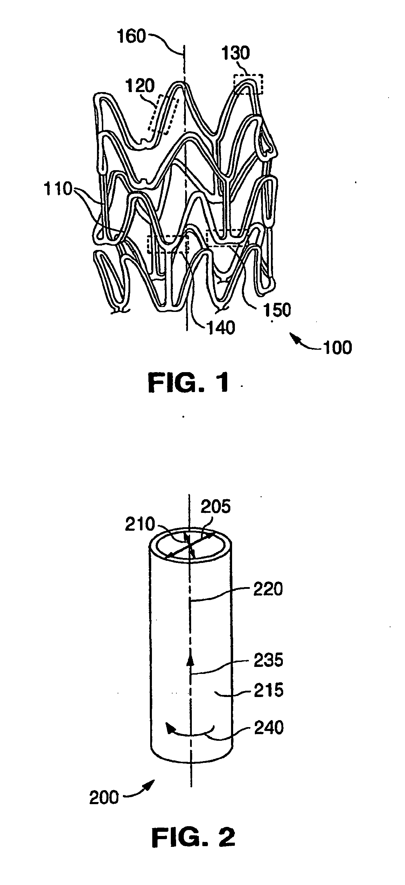

A stent can have a scaffolding or a substrate that includes a pattern of a plurality of interconnecting structural elements or struts. FIG. 1 depicts an example of a view of a stent 100. Stent 100 has a cylindrical shape with an axis 160 and includes a pattern with a number of interconnecting structural elements or struts 110. In general, a stent pattern is designed so that the stent can be radially compressed (crimped) and radially expanded (to allow deployment). The stresses involved during compression and expansion are generally distributed throughout various structural elements of the stent pattern. The present invention is not limited to the stent pattern depicted in FIG. 1. The variation in stent patterns is virtually unlimited.

The underlying structure or substrate of a stent can be completely or at least in part made from a biodegradable polymer or combination of biodegradable polymers, a biostable polymer or combination of biostable polymers, or a combination of biodegradable and biostable polymers. Additionally, a polymer-based coating for a surface of a device can be a biodegradable polymer or combination of biodegradable polymers, a biostable polymer or combination of biostable polymers, or a combination of biodegradable and biostable polymers.

A stent such as stent 100 may be fabricated from a polymeric tube or a sheet by rolling and bonding the sheet to form a tube. For example, FIG. 2 depicts a tube 200. Tube 200 is a cylindrically-shaped with an outside diameter 205 and an inside diameter 210. FIG. 2 also depicts a surface 215 and a cylindrical axis 220 of tube 200. In some embodiments, the diameter of the polymer tube prior to fabrication of an implantable medical device may be between about 0.2 mm and about 5.0 mm, or more narrowly between about 1 mm and about 3 mm. Polymeric tubes may be formed by various types of methods, including, but not limited to extrusion or injection molding.

A stent pattern may be formed on a polymeric tube by laser cutting a pattern on the tube. Representative examples of lasers that may be used include, but are not limited to, excimer, carbon dioxide, and YAG. In other embodiments, chemical etching may be used to form a pattern on a tube.

The pattern of stent 100 in FIG. 1 varies throughout its structure to allow radial expansion and compression and longitudinal flexure. A pattern may include portions of struts that are straight or relatively straight, an example being a portion 120. In addition, patterns may include bending elements 130, 140, and 150.

Bending elements bend inward when a stent is crimped to allow radial compression. Bending elements also bend outward when a stent is expanded to allow for radial expansion. After deployment, a stent is under static and cyclic compressive loads from the vessel walls. Thus, bending elements are subjected to deformation during use. "Use" includes, but is not limited to, manufacturing, assembling (e.g., crimping stent on a catheter), delivery of stent into and through a bodily lumen to a treatment site, and deployment of stent at a treatment site, and treatment after deployment.

Additionally, stent 100 is subjected to flexure along axis 160 when it is maneuvered through a tortuous vascular path during delivery. Stent 100 is also subjected to flexure when it has to conform to a deployment site that may not be linear.

Thus, a stent must have adequate strength in the radial direction to withstand structural loads, namely radial compressive forces, imposed on the stent as it supports the walls of a vessel. Radial strength is associated with strength of the stent around the circumferential direction of the stent. In addition, the stent must possess sufficient flexibility as well as strength along its longitudinal axis to allow for crimping, expansion, and flexure during delivery and after deployment. High strength is more significant in the radial direction, while both strength and flexibility are important axially. Therefore, the mechanical requirements of a stent differ in the axial and radial directions.

A significant advantage of polymeric stents is that they can be fabricated from biodegradable polymers. Thus, a biodegradable stent can be configured erode away from an implant site when it is no longer needed. However, polymers tend to have a number of shortcomings for use as materials for stents. Compared to metals, the strength to weight ratio of polymers is smaller than that of metals. A polymeric stent with inadequete radial strength can result in mechanical failure or recoil inward after implantation into a vessel. To compensate for the lower strength, a polymeric stent requires significantly thicker struts than a metallic stent, which results in an undesirably large profile.

However, it is well known by those skilled in the art that the mechanical properties of a polymer can be modified by applying stress to a polymer. James L. White and Joseph E. Spruiell, Polymer and Engineering Science, 1981, Vol. 21, No. 13. The application of stress can induce molecular orientation along the direction of stress which can modify mechanical properties along the direction of applied stress. For example, strength and modulus are some of the important properties that depend upon orientation of polymer chains in a polymer. Molecular orientation refers to the relative orientation of polymer chains along a longitudinal or covalent axis of the polymer chains.

A polymer may be completely amorphous, partially crystalline, or almost completely crystalline. A partially crystalline polymer includes crystalline regions separated by amorphous regions. The crystalline regions do not necessarily have the same or similar orientation of polymer chains. However, a high degree of orientation of crystallites may be induced by applying stress to a semi-crystalline polymer. The stress may also induce orientation in the amorphous regions. A high degree of molecular orientation can be induced even in an amorphous region. An oriented amorphous region also tends to have high strength and high modulus along an axis of alignment of polymer chains. Additionally, for some polymers under some conditions, induced alignment in an amorphous polymer may be accompanied by crystallization of the amorphous polymer into an ordered structure. This is known as stress induced crystallization.

As indicated above, due to the magnitude and directions of stresses imposed on a stent during use, it is important for the mechanical stability of the stent to have suitable mechanical properties, such as strength and modulus, in the axial and circumferential directions. Therefore, it can be advantageous to modify the mechanical properties of a tube, to be used in the fabrication of a stent, by induced orientation from applied stress in the axial direction, circumferential direction, or both. Thus, a modified tube can have a desired degree of orientation in both directions or biaxial orientation.

Polymer tubes formed by extrusion methods tend to possess a significant degree of axial polymer chain alignment. However, such conventionally extruded tubes tend to possess no or substantially no polymer chain alignment in the circumferential direction. A tube made from injection molding has a relatively low degree of polymer chain alignment in both the axial and circumferential directions.

Since highly oriented regions in polymers tend to be associated with higher strength and modulus, it may be desirable to incorporate processes that induce alignment of polymer chains along one or more preferred axes or directions into fabricating of stents.

Therefore, it can be desirable to fabricate a stent from a polymeric tube with induced orientation in the axial direction, as shown by an arrow 235 in FIG. 2 and in the circumferential direction as indicated by an arrow 240. A biaxial oriented tube may be configured to have desired strength and modulus in both the circumferential and axial directions.

In general, the expansion and extension of a tube are not independent. For instance, in one case, when a polymer tube is radially deformed or expanded in the absence of an axial tensile force, the axial length may tend to decrease. Similarly, in another case, when a polymer tube is axially deformed or extended in the absence of a radial force, radial shrinkage may occur, i.e., the diameter of the tube may tend to decrease. In both cases, the thickness of the tube can decrease. Whether the radial thickness decreases or increases depends on the rate of deformation and the force applied to deform the tube. For example, a relatively high deformation rate and/or force can reduce radial thickness with less radial shrinkage. In addition, when the degree of radial deformation is higher than the degree of axial deformation, the radial thickness may tend to decrease. The degree of radial and axial deformation may be given by radial and axial draw ratios, respectively, which are defined below.

The degree of radial expansion, and thus induced radial orientation and strength, of a tube can be quantified by a radial expansion (RE) ratio:

Outside Diameter of Expanded Tube

Original Inside Diameter of Tube

The RE ratio can also be expressed as a percent expansion:

$$\% \text{ Radial expansion} = (\text{RE ratio } -1) \times 100\%$$

Similarly, the degree of axial extension, and thus induced axial orientation and strength, may be quantified by an axial extension (AE) ratio:

Length of Extended Tube

Original Length of Tube

The AE ratio can also be expressed as a percent expansion:

$$\% \text{ Axial expansion} = (\text{AE ratio } -1) \times 100\%$$

The degree of polymer chain alignment induced with applied stress may depend upon the temperature of the polymer. Above $T_g$, polymer chain alignment may be readily induced with applied stress since polymer chains have greater mobility than below $T_g$. Consequently, the amount of deformation depends on the temperature of a polymeric material. Therefore, it is advantageous to deform the tube at a temperature above $T_g$.

The polymeric tube can be heated prior to and/or contemporaneously with deformation above $T_g$. The temper-

ature of the tube can be increased to a deformation temperature prior to deformation and maintained at the deformation temperature during deformation. The temperature of the tube can also be increased at a constant or nonlinear rate during deformation.

Additionally, the polymeric tube may be heat set after deformation to allow polymeric chains to rearrange upon deformation. "Heat setting" refers to allowing polymer chains to equilibrate or rearrange to the induced oriented structure, caused by the deformation, at an elevated temperature. Heat setting can be necessary since rearrangement of polymer chains is a time and temperature dependent process. An oriented structure that is thermodynamically stable at a given temperature may not be formed instantaneously. Thus, the structure may be formed over a period of time. During this time period, the polymer in the deformed state may be maintained at an elevated temperature to allow polymer chains to adopt the oriented structure. The polymer may be maintained in the deformed state by maintaining a radial pressure and axial tension in the tube.

In addition, the deformed tube may then be cooled. The tube can be cooled slowly from above Tg to below Tg. Alternatively, the tube can be cooled quickly or quenched below Tg to an ambient temperature. The tube can be maintained at the deformed diameter during cooling.

Additionally, the temperature of the deformation process is used to control the crystallinity of the deformation process. Although there are a number of physical properties of a polymer that affect the mechanical properties of a polymeric tube, crystallinity of the polymer of the tube is of great significance since the deformation process can influence the degree of crystallinity. In general, as the crystallinity of a polymer increases, the modulus of the polymer increases. Also, increasing crystallinity can also increase the Tg of a polymer, causing a polymer to exhibit brittle behavior at higher temperatures.

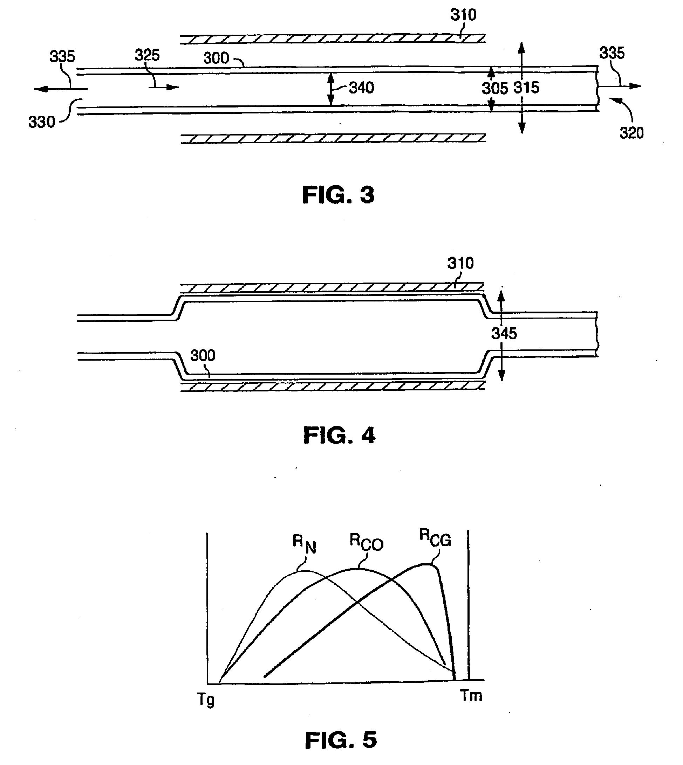

Therefore, the crystallinity of a polymer tube prior to deformation and the process conditions of the deformation can influence the mechanical properties in the axial and radial directions. The crystallinity of a deformed tube can be controlled by controlling the temperature of the deformation process. In general, crystallization tends to occur in a polymer at temperatures between Tg and Tm of the polymer. The rate of crystallization in this range varies with temperature. FIG. 5 depicts a schematic plot of the crystal nucleation rate ($R_N$), the crystal growth rate ($R_{CG}$), and the overall rate of crystallization ($R_{CO}$). The crystal nucleation rate is the growth rate of new crystals and the crystal growth rate is the rate of growth of formed crystals. The overall rate of crystallization is the sum of curves $R_N$ and $R_{CG}$.

The temperature of the tube during deformation is controlled to have a crystallization rate that results in a desired degree of crystallization. In some embodiments, the temperature can be in a range at which the overall crystallization rate is relatively low to eliminate or reduce an increase in crystallinity during deformation. In other embodiments, the temperature can be in a range at which the overall crystallization rate is relatively high to increase crystallinity during deformation. Additionally, crystallinity can also be controlled by controlling the temperature during heat setting.

The temperature is in a range in which the crystal nucleation rate is larger than the crystal growth rate. For example, the temperature can be where the ratio of the crystal nucleation rate to crystal growth rate is 2, 5, 10, 50, 100, or greater than 100. Under these conditions, the resulting polymer has a relatively large number of crystalline domains that are relatively small. As the size of the crystalline domains decreases along with an increase in the number of domains, the fracture toughness of the polymer can be increased without the onset of brittle behavior.

In some embodiments, a method of fabricating a stent can include selecting a value of a mechanical property along the longitudinal axis and/or the circumferential direction of a polymer tube. For example, a stent having particular strength or modulus along its axis may be desirable. The selected or desired value of mechanical property depends upon the mechanical requirements of a stent for a particular treatment. The method may further include axially extending and radially expanding the polymeric tube so that the tube has the value of the mechanical property along the longitudinal axis and/or the circumferential direction of a polymer tube. A stent with the desired mechanical property(ies) may then be fabricated from the tube.

Generally, the mechanical properties of an expanded and extended tube depend upon the radial expansion ratio, axial extension ratio, initial properties of the tube, and processing parameters of the deformation process. Certain embodiments of a method for fabricating stent can include determining an axial extension ratio and a radial expansion ratio of a polymeric tube that result in a selected or desired value of a mechanical property along the longitudinal axis and/or the circumferential direction of an axially extended and radially expanded tube.

The mechanical properties of a deformed tube, in particular along the axial or circumferential direction, also depend on the initial properties of the tube and the deformation processing parameters. Thus, the determined axial extension ratio and/or radial expansion ratio correspond to particular deformation process conditions and the initial properties of the tube.

Processing conditions can include, but are not limited to the temperature history of the tube during deformation, strain rate of the axial extension and the radial expansion, and time of the axial extension and radial expansion. The temperature of the tube can be constant during the deformation or be a function of time during the deformation process. Deformation processing conditions can also include the conditions during heat setting of the tube which can include the temperature history of the tube during heat setting.

Initial properties include, but are not limited to, the mechanical properties along axial and circumferential directions. Initial properties also include the degree of crystallinity. The initial properties of a polymeric tube are dependent upon such factors as the method of formation, such as extrusion or injection molding, and formation conditions.

Additionally, after determining an axial extension ratio and a radial expansion ratio of a polymeric tube, the method may then include axially extending and/or radially expanding a polymeric tube such that the axially extended and radially expanded tube has the determined axial draw ratio and radial blow-up ratio. A stent may then be fabricated from the axially extended and radially expanded tube. The fabricated stent can have the desired or selected value(s) of the mechanical property(ies) in the axial and/or circumferential direction.

Other embodiments of a method of fabricating a stent can include determining an axial extension ratio and/or a radial expansion ratio of a polymeric tube and a value of a deformation processing parameter that results in a selected or desired value of a mechanical property along the longitudinal axis and/or the circumferential direction of a tube. A polymeric tube may then be axially extended and/or radially expanded using the value of the deformation processing parameter to have the axial extension ratio and radial expansion ratio. A stent may then be fabricated from the tube.

In some embodiments, a polymeric tube may be deformed by blow molding. In blow molding, a tube can be deformed radially by increasing a pressure in the tube by conveying a fluid into the tube. The polymer tube may be deformed axially by applying a tensile force by a tension source at one end while holding the other end stationary. Alternatively, a tensile force may be applied at both ends of the tube. The tube may be axially extended before, during, and/or after radial expansion.

In some embodiments, blow molding may include first positioning a tube in a cylindrical member or mold. The mold may act to control the degree of radial deformation of the tube by limiting the deformation of the outside diameter or surface of the tube to the inside diameter of the mold. The inside diameter of the mold may correspond to a diameter less than or equal to a desired diameter of the polymer tube. Alternatively, the fluid temperature and pressure may be used to control the degree of radial deformation by limiting deformation of the inside diameter of the tube as an alternative to or in combination with using the mold.

As indicated above, the temperature of the tube can be heated to temperatures above the Tg of the polymer during deformation. The polymer tube may also be heated prior to, during, and subsequent to the deformation. In one embodiment, the tube may be heated by conveying a gas above ambient temperature on and/or into the tube. The gas may be the same gas used to increase the pressure in the tube. In another embodiment, the tube may be heated by translating a heating element or nozzle adjacent to the tube. In other embodiments, the tube may be heated by the mold. The mold may be heated, for example, by heating elements on, in, and/or adjacent to the mold.

Certain embodiments may include first sealing, blocking, or closing a polymer tube at a distal end. The end may be open in subsequent manufacturing steps. The fluid, (conventionally a gas such as air, nitrogen, oxygen, argon, etc.) may then be conveyed into a proximal end of the polymer tube to increase the pressure in the tube. The pressure of the fluid in the tube may act to radially expand the tube.

Additionally, the pressure inside the tube, the tension along the cylindrical axis of the tube, and the temperature of the tube may be maintained above ambient levels for a period of time to allow the polymer tube to be heat set. Heat setting may include maintaining a tube at a temperature greater than or equal to the Tg of the polymer and less than the Tm of the polymer for a selected period to time. The selected period of time may be between about one minute and about two hours, or more narrowly, between about two minutes and about ten minutes.

In heat setting, the polymer tube may then be cooled to below its Tg either before or after decreasing the pressure and/or decreasing tension. Cooling the tube helps insure that the tube maintains the proper shape, size, and length following its formation. Upon cooling, the deformed tube retains the length and shape imposed by an inner surface of the mold.

FIGs. 3 and 4 further illustrate an embodiment of deforming a polymer tube for use in manufacturing an implantable medical device, such as a stent. FIG. 3 depicts an axial cross-section of a polymer tube 300 with an outside diameter 305 positioned within a mold 310. Mold 310 may act to limit the radial deformation of polymer tube 300 to a diameter 315, the inside diameter of mold 305. Polymer tube 300 may be closed at a distal end 320. Distal end 320 may be open in subsequent manufacturing steps. A fluid may be conveyed, as indicated by an arrow 325, into an open proximal end 330 of polymer tube 300. A tensile force 335 is applied at proximal end 330 and a distal end 320.

Polymer tube 300 may be heated by heating the gas to a temperature above ambient temperature prior to conveying the gas into polymer tube 300. Alternatively, the polymer tube may be heated by heating the exterior of mold 310. The tube may also be heated by the mold. The increase in pressure inside of polymer tube 300, facilitated by an increase in temperature of the polymer tube, causes radial deformation of polymer tube 300, as indicated by an arrow 340. FIG. 4 depicts polymer tube 300 in a deformed state with an outside diameter 345 within mold 160.

Determining an axial extension ratio and a radial expansion ratio of a polymeric tube that result in a selected or desired value of a mechanical property along the longitudinal axis and/or the circumferential direction of an axially extended and radially expanded tube may be performed in a variety ways. The radial and axial draw ratios may be found empirically either through experiment or modeling.

In one embodiment, a set of tubes having the same or substantially the same properties may be fabricated or obtained. Two or more of the tubes may each be axially extended and radially expanded at different axial extension ratios and radial expansion ratios. Selected mechanical properties along the axial and radial direction can then be measured and compared to selected or desired mechanical properties.

In this way, the relationship between the deformation ratios and mechanical properties can be determined. Additionally, the relationship between a deformation processing condition and a mechanical property may be determined by deforming two or more tubes using different values of the processing condition. In a similar manner, modeling techniques such as finite element analysis may be employed to determine the relationship between deformation ratios and processing conditions to mechanical properties.

Polymers can be biostable, bioabsorbable, biodegradable or bioerodable. Biostable refers to polymers that are not biodegradable. The terms biodegradable, bioabsorbable, and bioerodable are used interchangeably and refer to polymers that are capable of being completely degraded and/or eroded when exposed to bodily fluids such as blood and can be gradually resorbed, absorbed, and/or eliminated by the body. The processes of breaking down and eventual absorption and elimination of the polymer can be caused by, for example, hydrolysis, metabolic processes, bulk or surface erosion, and the like.

It is understood that after the process of degradation, erosion, absorption, and/or resorption has been completed, no part of the stent will remain or in the case of coating applications on a biostable scaffolding, no polymer will remain on the device. In some embodiments, very negligible traces or residue may be left behind. For stents made from a biodegradable polymer, the stent is intended to remain in the body for a duration of time until its intended function of, for example, maintaining vascular patency and/or drug delivery is accomplished.

Representative examples of polymers that may be used to fabricate or coat an implantable medical device include, but are not limited to, poly(N-acetylglucosamine) (Chitin), Chitosan, poly(hydroxyvalerate), poly(lactide-co-glycolide), poly(hydroxybutyrate), poly(hydroxybutyrate-co-valerate), polyorthoester, polyanhydride, poly(glycolic acid), poly(glycolide), poly(L-lactic acid), poly(L-lactide), poly(D,L-lactic acid), poly(D,L-lactide), poly(caprolactone), poly(trimethylene carbonate), polyester amide, poly(glycolic acid-co-trimethylene carbonate), co-poly(ether-esters) (e.g. PEO/PLA), polyphosphazenes, biomolecules (such as fibrin, fibrinogen, cellulose, starch, collagen and hyaluronic acid), polyurethanes, silicones, polyesters, polyolefins, polyisobutylene and ethylene-alphaolefin copolymers, acrylic polymers and copolymers other than polyacrylates, vinyl halide polymers and copolymers (such as polyvinyl chloride), polyvinyl ethers (such as polyvinyl methyl ether), polyvinylidene halides (such as polyvinylidene chloride), polyacrylonitrile, polyvinyl ketones, polyvinyl aromatics (such as polystyrene), polyvinyl esters (such as polyvinyl acetate), acrylonitrile-styrene copolymers, ABS resins, polyamides (such as Nylon 66 and polycaprolactam), polycarbonates, polyoxymethylenes, polyimides, polyethers, polyurethanes, rayon, rayon-triacetate, cellulose, cellulose acetate, cellulose butyrate, cellulose acetate butyrate, cellophane, cellulose nitrate, cellulose propionate, cellulose ethers, and carboxymethyl cellulose. Another type of polymer based on poly(lactic acid) that can be used includes graft copolymers, and block copolymers, such as AB block-copolymers ("diblock-copolymers") or ABA block-copolymers ("triblock-copolymers"), or mixtures thereof.

Additional representative examples of polymers that may be especially well suited for use in fabricating or coating an implantable medical device include ethylene vinyl alcohol copolymer (commonly known by the generic name EVOH or by the trade name EVAL), poly(butyl methacrylate), poly(vinylidene fluoride-co-hexafluororpropene) (e.g., SOLEF 21508, available from Solvay Solexis PVDF, Thorofare, NJ), polyvinylidene fluoride (otherwise known as KYNAR, available from ATOFINA Chemicals, Philadelphia, PA), ethylene-vinyl acetate copolymers, and polyethylene glycol.

EXAMPLES

The examples and experimental data set forth below are for illustrative purposes only and are in no way meant to limit the invention. The following examples are given to aid in understanding the invention, but it is to be understood that the invention is not limited to the particular materials or procedures of examples. In particular the examples illustrate the influence of the axial extension ratio and axial expansion ratio on the mechanical properties of a polymeric tube. The examples also illustrate how to determine the relationship between the deformation ratios and the mechanical properties of a tube along the axial and circumferential directions.

Tables 1 and 2 provide measurements of mechanical properties of two lots of deformed extruded poly(lactide) tubes. The initial inside diameter of the tubes was 0.036 in. The deformation process conditions were the same for each of the tubes. The tubes were deformed using a blow molding process described above. Tensile testing of the samples was performed on a Instron model 5544. Lot # refers to the extrusion lot.

The initial crystallinity of the as extruded tubing was approximately 15 %, as measured by differential scanning calorimetry (DSC). The process gas temperature for the blow molding process was set just above the minimum value necessary to fully expand the polymer tubing.

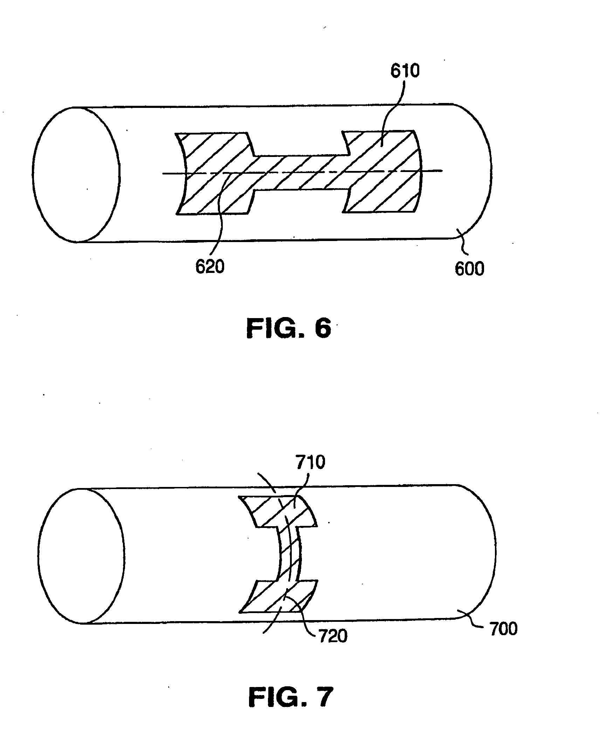

Table 1 provides measurements of dogbone samples taken from along the axial direction of a tube, as depicted in FIG. 6. FIG. 6 depicts a tube 600 showing an exemplary axial dogbone sample 610 from the surface of tube 600.

Mechanical properties were measured along line 620 of axial dogbone sample 610. The data in Table 2 shows that the strain to failure in the axial direction can be altered from 9% to 295% and the modulus in the axial direction can be varied from 370 ksi to 470 ksi. The strength or stress at peak varies between 8517 psi to 13,224 psi.

Table 1. Measurements of mechanical properties along the axial direction of tube from axial dogbone samples.

| Sample | Lot # | Axial Ext % | Radial Exp % | Crystallinity % | Stress at Break (psi) | Stress at Peak (psi) | Modulus (ksi) | Strain at Break (%) |
|---|---|---|---|---|---|---|---|---|
| 1 | 80 | 0 | 100 | 15 | 7009 | 8739 | 393 | 295 |
| 2 | 78 | 90 | 90 | 40 | 12603 | 13224 | 441 | 82 |
| 3 | 78 | 40 | 90 | 17.5 | 9051 | 9612 | 370 | 112 |
| 4 | 80 | 0 | 100 | 15 | 6985 | 8642 | 476 | 280 |
| 5 | 80 | 0 | 100 | 26 | 7687 | 8517 | 445 | 272 |
| 6 | 80 | 0 | 100 | 40 | 8297 | 10569 | 380 | 9 |

Table 2 provides measurements of dogbone samples taken along the circumferential direction of a tube, as depicted in FIG. 7. FIG. 7 depicts a tube 700 showing an axial dogbone 710 from the surface of tube 700. Mechanical properties were measured along line 720 of axial dogbone sample 710. The strain to failure in the radial direction can be altered from 112% to 130% and the modulus in the radial direction varies between 237 ksi to 340 ksi. The strength or stress at peak varies between 7290 psi to 8442 psi.

Table 2. Measurements of mechanical properties along the radial direction of tube from radial dogbone samples.

| Sample | Lot # | Axial Ext % | Radial Exp % | Crystallinity % | Stress at Break (psi) | Stress at Peak (psi) | Modulus (ksi) | Strain at Break (%) |
|---|---|---|---|---|---|---|---|---|
| 1 | 78 | 90 | 90 | 40 | 7627 | 8442 | 237 | 130 |
| 2 | 80 | 0 | 100 | 15 | 6300 | 7344 | 326 | 127 |
| 3 | 80 | 0 | 100 | 26 | 6582 | 7290 | 340 | 127 |
| 4 | 80 | 0 | 100 | 40 | 6940 | 7584 | 261 | 112 |

While particular embodiments of the present invention have been shown and described, it will be obvious to those skilled in the art that changes and modifications can be made without departing from this invention in its broader aspects.

**Claims**

1. A method for fabricating stent comprising:

   determining an axial extension ratio and a radial expansion ratio of a polymeric tube and a value of a deformation processing parameter that results in a selected value of a mechanical property along the longitudinal axis and/or the circumferential direction of an axially extended and/or radially expanded tube;
   selecting a temperature for the polymeric tube as the deformation processing parameter, so that the nucleation rate is at least twice the crystal growth rate of the polymer when the polymer is deformed, wherein the selected temperature is chosen so that when the tube is axially extended and radially expanded at the selected temperature, there is a decrease in the size of crystalline domains and increase in the number of crystalline domains formed in the strained tube;
   axially extending and radially expanding the polymeric tube at the selected temperature such that the axially extended and radially expanded tube has the desired axial draw ratio, radial blow-up ratio and nucleation rate to crystal growth rate to form a tube having improved fracture toughness; and
   fabricating a stent from the axially extended and radially expanded tube.

2. The method of claim 1, wherein the mechanical property is selected from the group consisting of strain at break, stress at break, stress at peak, and tensile modulus.

**3.** The method of claim 1, wherein the polymer comprises a biostable polymer, biodegradable polymer, or a combination thereof.

**4.** The method of claim 1, wherein the tube is radially expanded using blow molding.

**Patentansprüche**

**1.** Ein Verfahren zur Herstellung eines Stents umfassend:

das Ermitteln eines Axialerstreckungsverhältnisses und eines Radialausdehnungsverhältnisses eines polymeren Rohres und eines Wertes eines Verformungsverfahrensparameters, welcher einen ausgewählten Wert einer mechanischen Eigenschaft entlang der Längsachse und/oder der Umfangsrichtung eines axial erstreckten und/oder eines radial ausgedehnten Rohres zur Folge hat;
das Auswählen einer Temperatur für das polymere Rohr als der Verformungsverfahrensparameter, so dass die Keimbildungsgeschwindigkeit mindestens das Zweifache der Wachstumsgeschwindigkeit für die Kristallisation des Polymers beträgt, wenn das Polymer verformt wird, wobei die ausgewählte Temperatur so ausgewählt ist, dass, es zu einer Abnahme der Größe der kristallinen Domänen und zu einer Zunahme der Anzahl der im belasteten Rohr gebildeten kristallinen Domänen kommt, wenn das Rohr bei der ausgewählten Temperatur axial erstreckt und radial ausgedehnt wird;
das axiales Erstrecken und radiales Ausdehnen des polymeren Rohres bei der ausgewählten Temperatur, so dass das axial erstreckte und radial ausgedehnte Rohr das gewünschte axiale Reckverhältnis, radiale Aufblasverhältnis und Verhältnis der Keimbildungsgeschwindigkeig zur Wachstumsgeschwindigkeit für die Kristallisation hat, um ein Rohr mit verbesserter Bruchzähigkeit zu bilden; und
die Herstellung eines Stents aus dem axial erstreckten und radial ausgedehnten Rohr.

**2.** Das Verfahren des Anspruchs 1, wobei die mechanische Eigenschaft ausgewählt aus der Gruppe bestehend aus Bruchdehnung, Bruchspannung, Spitzenspannung und Zugmodul ist.

**3.** Das Verfahren des Anspruchs 1, wobei das Polymer ein biostabiles Polymer, ein biologisch abbaubares Polymer oder eine Kombination davon umfasst.

**4.** Das Verfahren des Anspruchs 1, wobei das Rohr mittels Blasformen radial ausgedehnt wird.

**Revendications**

**1.** Un procédé de fabrication d'un stent comprenant :

déterminer un taux d'extension axiale et un taux d'expansion radiale d'un tube polymère et une valeur d'un paramètre de traitement de déformation qui donne lieu à une valeur choisie d'une propriété mécanique le long de l'axe longitudinal et/ou de la direction circonférentielle d'un tube axialement étendu et/ou radialement élargi;
choisir une température pour le tube polymère en tant que le paramètre de traitement de déformation, de façon que la vitesse de nucléation soit au moins le double de la vitesse de croissance des cristaux du polymère lorsque le polymère est déformé, dans lequel la température sélectionnée est choisie de façon que lorsque le tube est axialement étendu et radialement élargi à la température sélectionnée, il y a une diminution de la grandeur des domaines cristallins et une augmentation du nombre de domaines cristallins formés dans le tube tendu;
étendre axialement et élargir radialement le tube polymère à la température sélectionnée de façon que le tube axialement étendu et radialement élargi ait le taux d'étirage axial désiré, le taux de soufflage et le rapport de la vitesse de nucléation à la vitesse de croissance de cristaux pour former un tube ayant une résistance aux fractures améliorée; et
fabriquer un stent à partir du tube axialement étendu et radialement élargi.

**2.** Le procédé de la revendication 1, dans lequel la propriété mécanique est choisie dans le groupe constitué de l'allongement à la rupture, de la traction à la rupture, de la limite de traction et du module de traction.

3. Le procédé de la revendication 1, dans lequel le polymère comprend un polymère biostable, un polymère biodégradable, ou une combinaison de ceux-ci.

4. Le procédé de la revendication 1, dans lequel le tube est radialement élargi moyennant soufflage.

**FIG. 1**

**FIG. 2**

**FIG. 3**

**FIG. 4**

**FIG. 5**

**FIG. 6**

**FIG. 7**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20060076708 A

**Non-patent literature cited in the description**

• **JAMES L. WHITE ; JOSEPH E. SPRUIELL.** *Polymer and Engineering Science,* 1981, vol. 21 (13